(19) Europäisches Patentamt
European Patent Office
Office européen des brevets

(11) Veröffentlichungsnummer: **0 433 806 B1**

(12) **EUROPÄISCHE PATENTSCHRIFT**

(45) Veröffentlichungstag der Patentschrift: **22.06.94**

(51) Int. Cl.[5]: **C07C 309/63**, C07C 303/30

(21) Anmeldenummer: **90123606.7**

(22) Anmeldetag: **08.12.90**

# (54) Isocyanatoalkylsulfonate und ein Verfahren zu deren Herstellung.

(30) Priorität: **22.12.89 DE 3942465**

(43) Veröffentlichungstag der Anmeldung:
**26.06.91 Patentblatt 91/26**

(45) Bekanntmachung des Hinweises auf die Patenterteilung:
**22.06.94 Patentblatt 94/25**

(84) Benannte Vertragsstaaten:
**CH DE FR GB LI**

(56) Entgegenhaltungen:
**EP-A- 0 131 200**
**EP-A- 0 132 734**
**WO-A-89/00589**
**US-A- 4 250 105**

**THE JOURNAL OF ORGANIC CHEMISTRY, Band 35, Nr. 9, September 1970, Seiten 3195-3196; R.K. CROSSLAND et al.: "A Facile Synthesis of Methanesulfonate Esters"**

**Chem. Abstr. Service "Registry Handbook" Number Section RN: 20570-49-4**

(73) Patentinhaber: **BAYER AG**
**D-51368 Leverkusen(DE)**

(72) Erfinder: **Schröder, Georg, Dr.**
**Akazienweg 19**
**W-5093 Burscheid(DE)**
Erfinder: **Arlt, Dieter, Prof. Dr.**
**Rybniker Strasse 2**
**W-5000 Koeln 80(DE)**
Erfinder: **Jautelat, Manfred, Dr.**
**Müllersbaum 28**
**W-5093 Burscheid 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bekannt, daß Sulfonsäureester aus den entsprechenden Alkoholen durch Veresterung mit Sulfonsäurechloriden [R.K. Crossland, K.L. Servis, J. Org. Chem., 35, 3195 (1970)] oder Sulfonsäureanhydriden [R.C. Paul, S.K. Sharma, R.D. Sharma, K.C. Malhotra, Chem. Ind., 1971, 702] zugänglich sind. Die bislang noch nicht bekannten Isocyanatoalkylsulfonate sollten sich auf diesem Wege nur schlecht herstellen lassen, da die zu deren Herstellung als Edukte benötigten Hydroxyalkylisocyanate in der Regel sehr labil sind [USP 4 250 105; F.W. Hoover, H.B. Stevenson, H.S. Rothrock, J. Org. Chem., 28, 1825 (1963)] und bei der Herstellung die Bildung von Chloralkylcarbamoylchloriden zu erwarten ist. Darüber hinaus muß bei der Herstellung [USP 4 250 105] mit einem Anteil von oligomerem Material gerechnet werden.

Allgemein sind alternative Verfahren zur Herstellung von Sulfonaten bekannt, so z.B. durch Reaktion von Halogeniden mit Silber- oder Kupfer-(I)-sulfonaten [P.W. Feit, O.T. Nelson, J. Med. Chem., 9, 416 (1966); JP 5973,587; CA 101, 130519]. Als möglicher Zugang zu den bislang noch nicht bekannten Isocyanatoalkylsulfonaten wurde der katalysierte nukleophile Austausch von Halogeniden gegen Sulfonate in Isocyanatoalkylhalogeniden gefunden. Die Austauschreaktion wurde bislang lediglich bei einfachen Alkylhalogeniden eingesetzt [R.C. Hahn, J. Org. Chem., 53, 5783 (1988)]. Aufgrund der hohen Reaktivität der Isocyanatgruppe mußte man befürchten, daß unter dem Einfluß der verwendeten Katalysatoren die Isocyanate trimerisieren würden [W. Broda, E.V. Dehmlow, H.J. Schulz, Isr. J. Chem., 26, 222 (1985)]. Überraschenderweise wurde gefunden, daß sich die Trimerisierung der Isocyanatgruppen unter den im folgenden geschilderten Bedingungen trotz Einfluß der Katalysatoren unterdrücken läßt und so die Isocyanatoalkylsulfonate herstellbar sind.

Gegenstand der vorliegenden Erfindung sind neue Isocyanatoalkylsulfonate der allgemeinen Formel

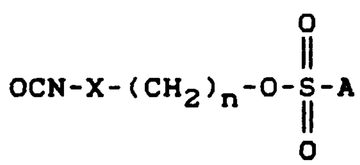

I

worin

- X für eine verzweigte oder unverzweigte Alkylengruppe mit 1-18 C-Atomen, eine Cycloalkylengruppe mit 5-14 C-Atomen, einen Arylenrest mit 6-20 C-Atomen, die gegebenenfalls durch $C_1$-$C_4$-Alkyl, $COOR^1$ oder $OR^2$ substituiert sein können, wobei $R^1$ und $R^2$ unabhängig voneinander für einen $C_1$-$C_4$-Alkylrest steht,
- n für eine ganze Zahl von 1-3,
- A für eine Alkylgruppe mit 1-18 C-Atomen, eine Cycloalkylgruppe mit 5-14 C-Atomen, einen Arylrest mit 6-20 C-Atomen oder eine Aralkylgruppe mit 7-16 C-Atomen steht, die gegebenenfalls durch eine Carbonamido-, Alkoxy-, Cyano- oder Carbonsäureestergruppe substituiert sein können, wobei das Sulfonatisocyanat der Formel

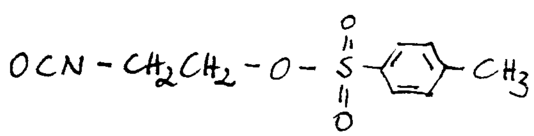

ausgenommen ist,

sowie ein Verfahren zur Herstellung von Isocyanatoalkylsulfonaten der allgemeinen Formel I, das dadurch gekennzeichnet ist, daß man ein Isocyanatoalkylhalogenid der allgemeinen Formel

$OCN\text{-}X\text{-}(CH_2)_n\text{-}Y$ (II)

worin X die in Formel I angegebene Bedeutung hat und

- Y für Chlor, Brom oder Jod steht,

mit einem Sulfonsäureester der allgemeinen Formel

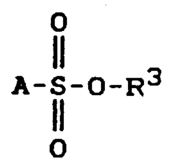

III

worin

A die in Formel I angegebene Bedeutung besitzt und

$R^3$ für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen steht,

umsetzt, in Gegenwart eines Katalysators der allgemeinen Formel

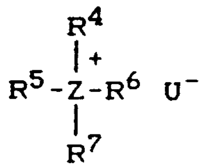 bzw. 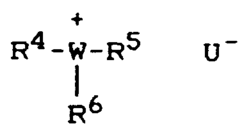 IV

IVa IVb

worin

$R^4$ bis $R^7$ unabhängig voneinander für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen stehen, oder

$R^4$ für den Rest eines Polymeren steht, welches an ein C-Atom der Polymerkette mindestens eine Gruppe

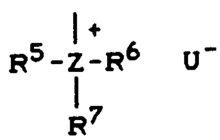 bzw. 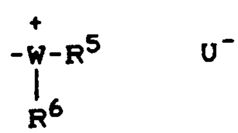

gebunden enthält,

Z für Stickstoff, Phosphor oder Arsen,

W für S oder S=O,

U für Cl, Br, J oder B, worin

B für die Formel $R^8$-$SO_3^{\ominus}$- und $R^8$ für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen steht.

Vorzugsweise setzt man 1 Mol II mit 0.1 bis 10 Mol III in Gegenwart von 0.001 bis 0.5 Mol IV bei etwa 50 bis 200 °C um.

Die Umsetzung erfolgt gegebenenfalls in Gegenwart eines inerten Verdünnungsmittels, z.B. N,N-Dimethylformamid, N,N-Dimethylacetamid, Dimethylsulfoxid, N-Methylpyrrolidon, Dimethoxyethan, Diethylenglykoldimethylether usw.. Die Reaktionszeiten liegen im allgemeinen bei mehreren Stunden in Abhängigkeit von Temperatur und Katalysatorkonzentration. Die Abtrennung von I erfolgt vorzugsweise durch Destillation, kann jedoch auch chromatographisch oder durch Kristallisation erfolgen. Es kann auch zweckmäßig sein im Verlaufe der Reaktion die Menge an Katalysator der allgemeinen Formel IV nachträglich schrittweise zu erhöhen.

Bevorzugte Verbindungen sind solche, in denen

X für eine Alkylengruppe mit 1 - 7 C-Atomen, eine Cycloalkylengruppe mit 5 - 6 C-Atomen, einen Arylenrest mit 6 - 10 C-Atomen, der gegebenenfalls durch einen $C_1$-$C_2$-Alkylrest, $COOR^1$ oder $OR^2$ substituiert sein kann,
wobei

$R^1$ und $R^2$ unabhängig voneinander für einen $C_1$-$C_4$-Alkylrest stehen,
n für eine Zahl von 1-2,
Y für Chlor oder Brom steht.

Als Ausgangsverbindungen seien im einzelnen genannt: 4-Chlor-isocyanatobutan, 5-Chlor-isocyanatopentan, 1-Chlor-2-isocyanatopropan, 2-Chlor-isocyanatopropan, 1-Chlor-2-isocyanatobutan, 3-Chlor-2,2-dimethylisocyanatopropan, 3-Chlor-2-isocyanatopropan, 3-Isocyanatojodpropan, 2-Brom-isocyanatoethan, 3-Brom-isocyanatopropan, 4-Brom-isocyanatobutan, 5-Brom-isocyanatopentan, 4-Chlor-methyl-phenylisocyanat, 3-Chlor-methyl-phenylisocyanat. Als besonders bevorzugt seien 2-Chlor-isocyanatoethan, 3-Chlor-isocyanatopropan und 6-Chlor-isocyanatohexan genannt.

Für Verbindungen der Formel III werden bevorzugt solche eingesetzt, worin
A und $R^3$ unabhängig voneinander für eine Alkylgruppe mit 1 - 6 C-Atomen, eine Cycloalkylgruppe mit 5 - 6 C-Atomen, einen Arylrest mit 6 - 10 C-Atomen oder eine Aralkylgruppe mit 7 - 10 C-Atomen stehen.

Im einzelnen seien genannt: Methansulfonsäurepropylester, Ethansulfonsäuremethylester, Ethansulfonsäureethylester, Ethansulfonsäurepropylester, Propansulfonsäuremethylester, Propansulfonsäureethylester, Propansulfonsäurepropylester, 4-Toluolsulfonsäuremethylester, 4-Toluolsulfonsäureethylester, 4-Toluolsulfonsäurepropylester, Benzolsulfonsäuremethylester, Benzolsulfonsäureethylester, Benzolsulfonsäurepropylester. Als besonders bevorzugt seien hier Methansulfonsäuremethylester und Methansulfonsäureethylester genannt.

Für Verbindungen der Formel IV werden bevorzugt solche eingesetzt, worin
$R^4$ bis $R^7$ unabhängig voneinander für eine Alkylgruppe mit 1 - 12 C-Atomen, eine Cycloalkylgruppe mit 5 - 10 C-Atomen, einen Arylrest mit 6 - 10 C-Atomen oder eine Aralkylgruppe mit 7 - 10 C-Atomen,

oder aber
$R^4$ für den Rest eines Polymeren steht, welches an ein C-Atom der Polymerkette mindestens eine Gruppe

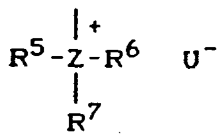 bzw. 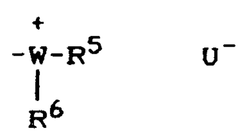

gebunden enthält,
Z für Stickstoff, Phosphor oder Arsen,
U für Chlor, Brom, Jod oder B, worin
B für die Formel $R^8$-$SO_3^{\ominus}$- und $R^8$ für eine Alkylgruppe mit 1 - 12 C-Atomen, eine Cycloalkylgruppe mit 5 - 10 C-Atomen, einen Arylrest mit 6 - 10 C-Atomen oder eine Aralkylgruppe mit 7 - 10 C-Atomen stehen.

Als Ausgangsverbindungen seien im einzelnen genannt: Tetramethylammoniumchlord, Tetramethylammoniumbromid, Tetramethylammoniumjodid, Tetramethylammoniummethansulfonat, Tetraethylammoniumchlorid, Tetraethylammoniumbromid, Tetraethylammoniumjodid, Tetraethylammoniummethansulfonat, Tetrapropylammoniumchlorid, Tetrapropylammoniumbromid, Tetrapropylammoniumjodid, Tetrapropylammoniumethansulfonat, Tetrabutylammoniumchlorid, Tetrahexylammoniumchlorid, Tetrahexylammoniumbromid, Tetrahexylammoniummethansulfonat, Tetrabutylphosphoniumchlorid, Tetrabutylphosphoniumjodid, Tetrabutylphosphoniummethansulfonat. Als besonders bevorzugt seien hier Tetrabutylammoniumbromid, Tetrabutylammoniumjodid, Tetrabutylammoniumethansulfonat, Tetrahexylammoniumjodid und Tetrabutylphosphoniumbromid aufgeführt.

Geeignete hochmolekulare Verbindungen IV sind beispielsweise durch Umsetzung von gegebenenfalls vernetzten (z.B. mit Divinylbenzol) chlormethylierten Styrol-Homopolymerisaten bzw. Styrol-Copolymerisaten mit Verbindungen der Formel

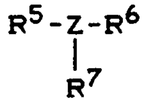 bzw. 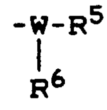

erhältlichen Produkte.

Die Isocyanatoalkylhalogenide sind bekannt bzw. lassen sich nach bekannten Verfahren herstellen, vgl. z.B. Houben-Weyl, Bd. E4, S 738 ff; W. Siefken, Liebigs Ann. Chem., 562,72 (1949); H. Wenker, J. Am. Chem. Soc., 58, 2608 (1936); O. Bayer, Angew. Chem., 59, 257 (1947); H. Najer, P. Chabrier, R. Guidicelli, Bull. Soc. Chim. Fr., 1959, 1611; DBP 947470; DE 3 326 874; K.D. Kampe, Liebigs Ann. Chem., 752, 142 (1971); A. Krantz, B. Hoppe, Tetrahedron Lett., 1975, 695; C.K. Johnson, J. Org. Chem., 32 (5), 1508, (1967); H.R. Kricheldorf, Angew. Chem., 91, 749 (1979).

Die neuen Verbindungen der allgemeinen Formel I finden beispielsweise Verwendung als Vernetzer bei der Herstellung von Polymeren. So lassen sich durch Umsetzung mit Polyamidaminen oder Polyaminen in Analogie zur Umsetzung mit anderen bifunktionellen Vernetzern, z.B. Epichlorhydrin, z.B. Papierausrüstungsmittel erhalten.

Geeignete Ausgangskomponenten und Verfahrensbedingungen sind beispielsweise in folgenden Literaturstellen beschrieben: DE-A 2 229 219, 1 771 043, 2 257 271, 2 938 588, 2 949 870, 1 720 905, 1 906 450, EP-A 0 131 200, 0 126 176, US-PS 2 926 154 und 3 332 901.

Beispiel 1

Darstellung von 2-Isocyanato-methansulfonyloxyethan

Eine Lösung von 36.0 g (0.34 Mol) 2-Chlorisocyanatoethan und 93.3 g (0.85 Mol) Methansulfonsäuremethylester werden nach Zugabe von 20.0 g (0.06 Mol) Tetrabutylammoniummethansulfonat für 6 h auf 120 °C erhitzt. Es wird am Dünnschichtverdampfer bei 180 °C und 0.04 mbar Druck abdestilliert und das Rohprodukt durch Destillation gereinigt. Man gewinnt so 18.0 g (0.17 Mol) 2-Chlorisocyanatoethan sowie 71.0 g (0.65 Mol) Methansulfonsäuremethylester zurück.

| | |
|---|---|
| Ausbeute: | 10.50 g (0.06 Mol, 35% d. Th.) |
| SDP: | 97 °C (0,05 bar) |
| $^1$H-NMR: | ($CDCl_3$, 200 MHz) $\delta$ = 3,10 (s; 3H), 2,66 (t, J = 5 Hz; 2H) 4,32 (t, J = 5 Hz; 2H) |
| $^{13}$C-NMR: | ($CDCl_3$, 50 HHz) $\delta$ = 37,65, 42,36, 68,18, 121,0 |
| IR: | 2260 (s), 1340 (s), 1165 (s), 1015 (s), 960 (s), 900 (s), 790 (s) |

Beispiel 2

Darstellung von 3-Isocyanato-methansulfonyloxypropan

Eine Lösung von 100.0 g (0.84 Mol) 3-Chlorisocyanatopropan und 92.4 g (0.84 Mol) Methansulfonsäuremethylester werden nach Zugabe von 28.4 g (0.084 Mol) Tetrabutylammoniummethansulfonat für 14 h auf 120 °C erhitzt.

Es wird am Dünnschichtverdampfer bei 180 °C und 0.04 mbar Druck abdestilliert und das Rohprodukt durch Destillation gereinigt. Man gewinnt so 22.85 g (0.21 Mol) Methansulfonsäuremethylester sowie 5.3 g (0.04 Mol) 3-Chlorisocyanatopropan zurück.

| | |
|---|---|
| Ausbeute: | 48.00 g (0.27 Mol, 33% d. Th.) |
| SDP: | 102-104 °C (0.04 mbar) |
| $^1$H-NMR: | ($CDCl_3$, 200 MHz) $\delta$ = 2,02 (q , J = 5 Hz; 2H), 3,10 (s; 3H); 3,55 (t, J = 5Hz; 2H); 4,35 (t; J = 5 Hz; 2H) |
| $^{13}$C-NMR: | ($CDCl_3$, 50 MHz) $\delta$ = 30,38, 37,22, 39,23, 66,69, 121,0 |
| IR: | 2260 (s), 1340 (s), 1165 (s), 101 (s), 970 (s), 940 (s) |

Beispiel 3

Darstellung von 6-Isocyanatomethansulfonyloxyhexan

Eine Lösung von 242.25 g (1.50 Mol) 6-Chlorisocyanatohexan und 165.00 g (1.50 Mol) Methansulfonsäuremethylester werden nach Zugabe von 50.0 g (0.15 Mol) Tetrabutylammoniummethansulfonat für 16 h auf 140 °C erhitzt.

Es wird über Kieselgel filtriert und mit Essigester/Cyclohexan (1:3; v:v) eluiert und das Rohprodukt durch Destillation gereinigt. Man gewinnt so 99.3 g (0.61 Mol) 6-Chlorisocyanatohexan zurück.

| | |
|---|---|
| Ausbeute: | 74.80 g (0.34 Mol, 38% d. Th.) |
| SDP: | 123-137 °C (0,1 mbar) |

$^1$H-NMR: (CDCl$_3$, 200 MHz) δ = 1,45 (mc; 4H), 163 (mc; 2H), 1,77 (mc; 2H), 3,01 (s; 3H), 3,33 (t, J = 5,5 Hz; 2H) 4,24 (t, J = 5,5 Hz; 2H)

$^{13}$C-NMR: (CDCl$_3$, 50 MHz) δ = 24,85, 25,98, 28,97, 30,99, 37,07, 42,84, 70,30, 121,96,

IR: 2940 (s), 2290 (s), 1360 (s), 1185 (s), 990 (s), 4965 (s), 930 (s).

## Patentansprüche

**1.** Sulfonatisocyanate der Formel

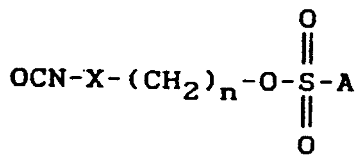

I

worin

X für eine verzweigte oder unverzweigte Alkylengruppe mit 1-18 C-Atomen, eine Cycloalkylengruppe mit 5-14 C-Atomen, einen Arylenrest mit 6-20 C-Atomen, die gegebenenfalls durch $C_1$-$C_4$-Alkyl, COOR$^1$ oder OR$^2$ substituiert sein können, wobei R$^1$ und R$^2$ unabhängig voneinander für einen $C_1$-$C_4$-Alkylrest steht,

n für eine ganze Zahl von 1-3,

A für eine Alkylgruppe mit 1-18 C-Atomen, eine Cycloalkylgruppe mit 5-14 C-Atomen, einen Arylrest mit 6-20 C-Atomen oder eine Aralkylgruppe mit 7-16 C-Atomen steht, die gegebenenfalls durch eine Carbonamido-, Alkoxy-, Cyano- oder Carbonsäureestergruppe substituiert sein können,

mit Ausnahame des Sulfonatisocyanats der Formel

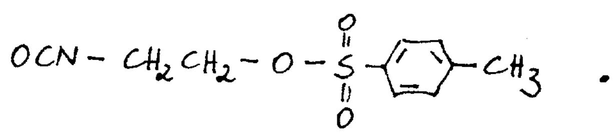

**2.** Verfahren zur Herstellung von Sulfonatisocyanaten der Formel

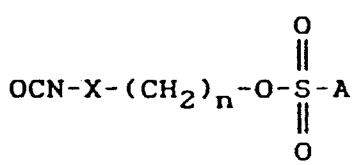

I

worin

X, A und n die in Anspruch 1 angegebene Bedeutung haben,

dadurch gekennzeichnet, daß man ein Isocyanathalogenid der Formel

$OCN-X-(CH_2)_n-Y$ (II)

worin X die in Anspruch 1 angegebene Bedeutung hat und

Y für Chlor, Brom oder Jod steht,

mit einem Sulfonsäureester der allgemeinen Formel

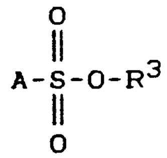

III

worin

A die in Anspruch 1 angegebene Bedeutung besitzt und

$R^3$ für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen steht,

in Gegenwart eines Katalysators der allgemeinen Formel

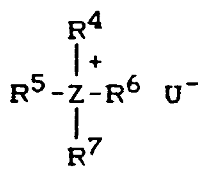

bzw.

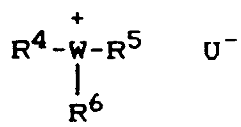

IVa IVb

worin

$R^4$ bis $R^7$ unabhängig voneinander für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen stehen, oder

$R^4$ für den Rest eines Polymeren steht, welches an ein C-Atom der Polymerkette mindestens eine Gruppe

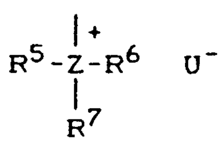

bzw.

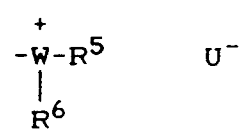

gebunden enthält,

Z für Stickstoff, Phosphor oder Arsen,

W für S oder S = O,

U für Cl, Br, J oder B

worin

B für die Formel $R^8$-$SO_3^{\ominus}$- und $R^8$ für eine Alkylgruppe mit 1-18 C-Atomen, eine Cycloalkylgruppe mit 5 - 14 C-Atomen, einen Arylrest mit 6 - 20 C-Atomen oder eine Aralkylgruppe mit 7 - 16 C-Atomen steht,

umsetzt.

**Claims**

**1.** Sulphonate/isocyanates of the formula

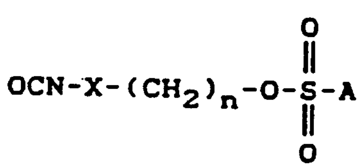

I

wherein

X represents a branched or unbranched alkylene group having 1-18 C atoms, a cycloalkylene group having 5-14 C atoms or an arylene radical having 6-20 C atoms, each of which can optionally be substituted by $C_1$-$C_4$-alkyl, $COOR^1$ or $OR^2$, wherein $R^1$ and $R^2$ independently of one another represent a $C_1$-$C_4$-alkyl radical,

n represents an integer from 1 to 3 and

A represents an alkyl group having 1-18 C atoms, a cycloalkyl group having 5-14 C atoms, an aryl radical having 6-20 C atoms or an aralkyl group having 7-16 C atoms, each of which can optionally be substituted by a carboxamido, alkoxy, cyano or carboxylic acid ester group, with the exception of the sulphonate/isocyanate of the formula

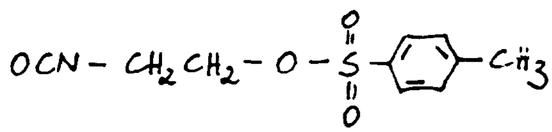

**2.** Process for the preparation of sulphonate/ isocyanates of the formula

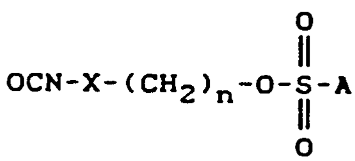

I

wherein

X, A and n have the meaning given in Claim 1,

characterized in that an isocyanate/halide of the formula

$OCN\text{-}X\text{-}(CH_2)_n\text{-}Y$ (II)

wherein X has the meaning given in Claim 1 and

Y represents chlorine, bromine or iodine,

is reacted with a sulphonic acid ester of the general formula

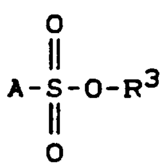

III

wherein

A has the meaning given in Claim 1 and

$R^3$ represents an alkyl group having 1-18 C atoms, a cycloalkyl group having 5-14 C atoms, an aryl radical having 6-20 C atoms or an aralkyl group having 7-16 C atoms,

in the presence of a catalyst of the general formula

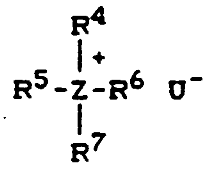 or 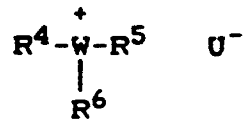

IVa IVb

wherein

- $R^4$ to $R^7$ independently of one another represent an alkyl group having 1-18 C atoms, a cycloalkyl group having 5-14 C atoms, an aryl radical having 6-20 C atoms or an aralkyl group having 7-16 C atoms, or
- $R^4$ represents the radical of a polymer which contains at least one group

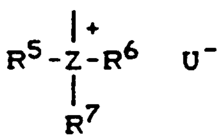 or 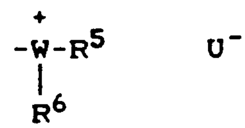

bonded to a C atom of the polymer chain,

- Z represents nitrogen, phosphorus or arsenic,
- W represents S or S = O,
- U represents Cl, Br, I or B,

wherein

- B represents the formula $R^8\text{-}SO_3^{\ominus}$- and $R^8$ represents an alkyl group having 1-18 C atoms, a cycloalkyl group having 5-14 C atoms, an aryl radical having 6-20 C atoms or an aralkyl group having 7-16 C atoms.

## Revendications

**1.** Isocyanatosulfonates de formule:

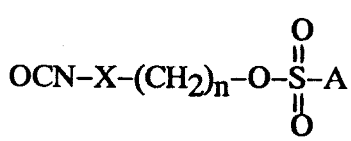 I

dans laquelle

X représente un groupe alkylène ramifié ou non ramifié en $C_1$-$C_{18}$, un groupe cycloalkylène en $C_5$-$C_{14}$, un résidu arylène en $C_6$-$C_{20}$, qui, le cas échéant, peuvent être substitués par un groupe alkyle en $C_1$-$C_4$, $COOR^1$ ou $OR^2$, $R^1$ et $R^2$ représentant indépendamment l'un de l'autre un résidu alkyle en $C_1$-$C_4$,

n représente un entier valant de 1 à 3,

A représente un groupe alkyle en $C_1$-$C_{18}$, un cycloalkyle en $C_5$-$C_{14}$, un résidu aryle en $C_6$-$C_{20}$ ou un groupe aralkyle en $C_7$-$C_{16}$, qui, le cas échéant, peuvent être substitués par un groupe carbonamide, un groupe alcoxyle, un groupe cyano ou un groupe ester d'acide carboxylique,

à l'exception de l'isocyanatosulfonate de formule:

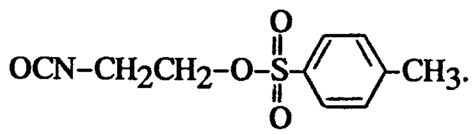

2. Procédé de production d'isocyanatosulfonates de formule:

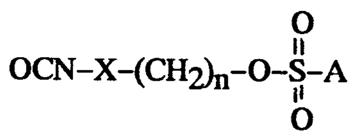 I

dans laquelle
X, A et n ont la signification donnée dans la revendication 1, caractérisé en ce qu'on fait réagir un halogénure d'isocyanate de formule:

$OCN-X-(CH_2)_n-Y$ (II)

dans laquelle
X a la signification donnée dans la revendication 1 et
Y représente le chlore, le brome ou l'iode,
avec un ester d'acide sulfonique de formule générale:

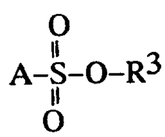 III

dans laquelle
A possède la signfication donnée dans la revendication 1 et
$R^3$ représente un groupe alkyle en $C_1$-$C_{18}$, un groupe cycloalkyle en $C_5$-$C_{14}$, un résidu aryle en $C_6$-$C_{20}$ ou un groupe aralkyle en $C_7$-$C_{16}$,
en présence d'un catalyseur de formule générale:

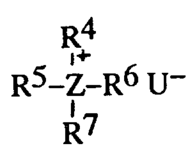

IVa

ou selon le cas

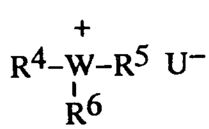

IVb

dans laquelle
$R^4$ à $R^7$ indépendamment l'un de l'autre représentent un groupe alkyle en $C_1$-$C_{18}$, un groupe

cycloalkyle en $C_5$-$C_{14}$, un résidu aryle en $C_6$-$C_{20}$ ou un groupe aralkyle en $C_7$-$C_{16}$, ou

$R^4$ représente le résidu d'un polymère, qui contient au moins un groupe

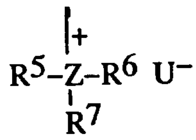

ou selon le cas

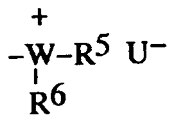

lié à un atome de carbone de la chaîne polymérique,

Z représente l'azote, le phosphore ou l'arsenic,

W représente S ou S = O,

U représente Cl, Br, I ou B

B représente la formule $R^8$-$SO_3^{\ominus}$ et $R^8$ représente un groupe alkyle en $C_1$-$C_{18}$, un groupe cycloalkyle en $C_5$-$C_{14}$, un résidu aryle en $C_6$-$C_{20}$ ou un groupe aralkyle en $C_7$-$C_{16}$.